# EUROPÄISCHE PATENTANMELDUNG

(11) **EP 4 385 340 A1**
(43) Veröffentlichungstag der Anmeldung: **19.06.2024**
(21) Anmeldenummer: 22213935.4
(22) Anmeldetag: 15.12.2022
(51) Int. Cl.: A23L 29/206, A23L 29/30, A23L 33/125, A23L 33/21, A23J 3/14

(54) **PREBIOTISCHE ZUBEREITUNGEN**

(71) Anmelder: DMK Deutsches Milchkontor GmbH, 27404 Zeven (DE)
(72) Erfinder: DÖRING, Sven-Rainer, 27404 Zeven (DE); Steffens, Marco, 27404 Elsdorf (DE)
(74) Vertreter: Fabry, Bernd

(57) **Zusammenfassung**

Vorgeschlagen werden prebiotische Zubereitungen, enthaltend oder bestehend aus
(a1) Milchproteinen und/oder
(a2) pflanzlichen Proteinen und
(b) Oligosacchariden ausgewählt aus der Gruppe, die gebildet wird von beta-Galactooligosacchariden (β-GOS), alpha-Galactooligosacchariden (α-GOS), Fructooligosacchariden (FOS) und Mannanoligosacchariden (MOS).

## Beschreibung

### GEBIET DER ERFINDUNG

Die vorliegende Erfindung befindet sich auf dem Gebiet der Milchprodukte und betrifft prebiotische Zubereitungen mit bestimmten Proteinen und bestimmten Kohlenhydraten.

### TECHNOLOGISCHER HINTERGRUND

Proteine, die mit der Nahrung aufgenommen werden, versorgen den Körper mit unentbehrlichen Aminosäuren und Stickstoff für den körpereigenen Aufbau von Eiweißen, z. B. Strukturproteine wie Actin, Myosin und Kreatin, Transportproteine wie Hämoglobin oder Transferrin, Rezeptorproteine, immunaktive Proteine wie Immunglobuline, und anderen stickstoffhaltigen Verbindungen, z. B. Enzyme, Peptidhormone wie Insulin sowie DNA und RNA. Aminosäuren sind außerdem Vorstufen in der Synthese von zahlreichen Stoffwechselprodukten wie z. B. Gallensäuren, Serotonin und Histamin.

Im menschlichen Körper werden 20 verschiedene Aminosäuren zum Aufbau von Proteinen benötigt, diese werden als proteinogen bezeichnet. Neun der proteinogenen Aminosäuren können im menschlichen Organismus nicht neu aufgebaut werden, sie werden als unentbehrlich (früher: essenziell) bezeichnet: Isoleucin, Leucin, Lysin, Methionin, Phenylalanin, Threonin, Tryptophan, Valin sowie für Säuglinge Histidin. Ohne eine regelmäßige Zufuhr dieser unentbehrlichen Aminosäuren können Mangelerscheinungen auftreten.

Die wichtigsten Proteinquellen sind seit jeher Fisch und Fleisch. In den letzten Jahren hat jedoch der Trend zu fleischloser Ernährung - sei es aus ethischen oder gesundheitlichen Gründen - stark zugenommen, was dazu geführt hat, dass Proteine aus alternativen Quellen an Bedeutung gewonnen haben. Hier sind vor allem Milchproteine zu nennen und in neuerer Zeit auch pflanzliche Proteine, zumal diese als vegan eingestuft werden. Nachteilig ist jedoch, dass die nicht-tierischen Proteine, insbesondere Pflanzenproteine, einen bitteren und mehr oder weniger starken Eigengeschmack aufweisen, den es zu überdecken gilt.

Gleichzeitig besteht im Markt das Bedürfnis nach Proteinprodukten, die über weitere Funktionalitäten verfügen, insbesondere im Bereich der Sportlernahrung.

### RELEVANTER STAND DER TECHNIK

EP 2124639 B1 (FRAUNHOFER) bezieht sich auf ein Verfahren zur Modifizierung des Geschmacksprofils einer pflanzlichen Proteinzubereitung, insbesondere einer Proteinzubereitung aus einer Hülsenfrucht. Das Proteinpräparat wird mit wasserlöslichen Kohlenhydraten in einer wässrigen Lösung in Kontakt gebracht, bevor es einem Lebensmittel zugesetzt wird, wobei der Kontakt das Geschmacksprofil von Proteinpräparaten aus Leguminosen vorteilhaft beeinflusst, so dass die Präparate in Lebensmitteln verwendet werden können, ohne deren Geschmack wesentlich zu verändern.

EP 0936875 B1 (NOVOZYMES) betrifft die Herstellung eines Aromastoffes für Lebensmittel. Insbesondere stellt die Erfindung ein Verfahren zur Herstellung eines Aromastoffs für Lebensmittel bereit, das die Schritte der Herstellung einer wässrigen Aufschlämmung von Pflanzenprotein und Pflanzenkohlenhydrat, der Behandlung der Aufschlämmung mit einer Protease, der Behandlung der Aufschlämmung mit einer Carbohydrase und der Reifung umfasst. Durch dieses Verfahren können verschiedene Geschmackseigenschaften im Proteinhydrolysat induziert werden.

### AUFGABE DER ERFINDUNG

Die Aufgabe der vorliegenden Erfindung hat daher darin bestanden, die beiden Bedürfnisse des Marktes, nämlich zum einen, nicht-tierische Proteinzubereitungen mit verbesserten Geschmackseigenschaften und zum anderen Proteinzubereitungen mit zusätzlichen Funktionalitäten, gleichzeitig mit einem neuen Produkt zu befriedigen. Die Zubereitungen sollten ferner so flexibel sein, dass sie den Wünschen unterschiedlicher Konsumentengruppen gerecht werden, also sowohl als vegetarisch, rein milchbasiert oder vegan eingestuft werden können.

### BESCHREIBUNG DER ERFINDUNG

Ein erster Gegenstand der Erfindung betrifft prebiotische Zubereitungen, enthaltend oder bestehend aus
(a1) Milchproteinen und/oder
(a2) pflanzlichen Proteinen und
(b) Oligosacchariden ausgewählt aus der Gruppe, die gebildet wird von beta-Galactooligosacchariden (β-GOS), alpha-Galactooligosacchariden (α-GOS), Fructooligosacchariden (FOS) und Mannanoligosacchariden (MOS).

Überraschenderweise wurde gefunden, dass Oligosaccharide der genannten Art eine ausgezeichnete Ergänzung zu Proteinkomponenten darstellen, weil sie diese zum einen prebiotisch machen und zum anderen den Nährwert erhöhen, ohne dass den Produkten Zucker zugesetzt werden muss. Die Oligosaccharide verleihen den Zubereitungen Textur/ Struktur und eine gewisse Süße und maskieren zusätzlich den zuweilen bitteren Eigengeschmack der Proteine. In diesem Zusammenhang sei darauf hingewiesen, dass die Maskierung eines Geschmackes nicht damit verwechselt werden darf, dass der Geschmack einfach durch die Menge eines anderen Stoffes überdeckt wird. Maskierung bedeutet, dass zwei Geschmacksrichtungen sich vereinigen und einen neuen Geschmack erzeugen; typisch ist hier, dass die Maskierung im Vergleich zur Überdeckung mit wesentlich geringeren Mengen auskommt.

Die Erfindung stellt zudem das gewünschte Baukastensystem zur Verfügung, bei dem aus zwei Produktgruppen - Proteinen und Oligosacchariden - und jeweils zwei Mitgliedern beliebig kombiniert werden kann. Eine Kombination aus Milchproteinen und β-GOS, die durch Transgalactosilierung von Milchzucker gewonnen wurden, ist beispielsweise vollständig Milch basiert. Eine Zubereitung, die pflanzliche Proteine und α-GOS auf Basis von Rohrzucker enthält, wäre hingegen vegan.

### Milchproteine

Milchprotein besteht aus den Komponenten Kasein (ca. 80 %) und Molkenprotein (ca. 20 %). Casein liegt in der Milch in phosphorylierter Form und mit Calcium assoziiert (Calcium-Proteinat-Phosphat-Partikel) in kolloidaler Verteilung (kolloid) vor. Es enthält alle essentiellen Aminosäuren, die auch bei der Be- und Verarbeitung der Milch nicht zerstört werden. Bei der Auftrennung von Casein erhält man die α-, β- und γ-Caseine, die ihrerseits in weitere Fraktionen zerlegt werden können. Eine wichtige Unterfraktion ist das κ-Casein, das in der Milch die Funktion eines Schutzkolloids ausübt.

Bei der Herstellung von Milchprotein wird auf die Trennung der beiden Eiweißkomponenten verzichtet. Stattdessen wird Kuhmilch als Basis genommen und das Protein herausgefiltert. Man erhält ein Milchproteinkonzentrat. Beim Milchproteinkonzentrat entfällt ein Großteil des im Milchpulver noch enthaltenen Fettes und Milchzuckers. Werden diese beiden Nährstoffe aus dem Konzentrat entfernt, bleibt lediglich der Eiweißanteil zurück, das sogenannte Milchproteinisolat.

Im Sinne der vorliegenden Erfindung können die Milchproteine ausgewählt sein aus der Gruppe, die gebildet wird von Casein, Milchproteinkonzentraten, Milchproteinisolaten, Molkenproteinen, Molkenproteinkonzentraten und proteinhaltigen Milchpulvern.

### Pflanzliche Proteine

Pflanzliche Proteine im Sinne der Erfindung (Komponente a) können aus Kartoffeln, Soja, Erbsen, Lupinen, Raps oder anderen proteinreichen Früchten wie z. B. Sonnenblumen- oder Kürbiskernen u. a. m. gewonnen werden. Die geernteten proteinhaltigen Früchte werden mechanisch zerkleinert und entfettet. Es entstehen Flocken oder ein proteinreiches Pulver. Anschließend wird unter Verwendung von Lösungsmitteln ein Proteinkonzentrat gewonnen, das ggf. weiter zu Proteinisolat gereinigt und aufkonzentriert wird: Die Flocken oder Mehl werden mit Wasser versetzt und angemaischt. Die proteinarmen Fasern und Feststoffe werden im nächsten Schritt mit Hilfe von Industriezentrifugen von der proteinreichen Lösung abgetrennt. Dann folgt die sogenannte Ausfällung. Hier wird der pH-Wert der proteinreichen Lösung auf den isoelektrischen Punkt eingestellt. Dadurch setzen sich die Proteinpartikel ab. Diese werden dann wiederum mittels Zentrifugen von der Lauge abgetrennt. Um alle Bestandteile der Mutterlauge aus dem ausgefällten und abgetrennten Protein zu entfernen, wird das Protein erneut mit Wasser versetzt und wieder mit Hilfe der Zentrifugalkraft abgetrennt. Bei einer Trockenextrusion wird unter Zuführung von Wärme, Druck und Hilfsstoffen ein Zwischenprodukt mit niedrigem Wassergehalt erzeugt. Diese ebenfalls geeigneten Proteine werden als TVP (Texturized Vegetable Protein) bezeichnet und haben eine trockene Konsistenz in Form von Körnern, Streifen oder Flocken. Bei einer Nassextrusion wird alternativ mit einem höheren Wassergehalt operiert. Die Feuchtigkeit des Zwischenproduktes liegt daher näher am Wassergehalt des Endproduktes. Das Zwischenprodukt wird als HMMA (High Moistured Meat Analogues) bezeichnet.

### Oligosaccharide

### Beta-Galactooligosaccharide (β-GOS) (Komponente b1)

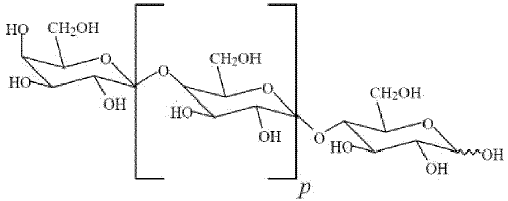

auch bekannt als Oligogalaktosyllactose, Oligogalaktose, Oligolaktose oder Transgalaktooligosaccharide (TOS), gehören zur Gruppe der Prebiotika. GOS kommt in handelsüblichen Produkten wie Nahrung für Säuglinge und Erwachsene vor. Aufgrund der Konfiguration ihrer glykosidischen Bindungen widerstehen Galactooligosaccharide (GOS) weitgehend der Hydrolyse durch Speichel- und Darmverdauungsenzyme. Galactooligosaccharide werden daher als Präbiotika eingestuft, definiert als unverdauliche Nahrungsbestandteile, die sich vorteilhaft auf den Wirt auswirken, indem sie das Wachstum und/oder die Aktivität nützlicher Bakterien im Dickdarm stimulieren. Die erhöhte Aktivität dieser gesundheitsfördernden Bakterien führt zu einer Reihe von Effekten, sowohl direkt durch die Bakterien selbst als auch indirekt durch die organischen Säuren, die sie durch Fermentation produzieren. Beispiele für Wirkungen sind die Stimulierung der Immunfunktionen, die Aufnahme essentieller Nährstoffe, und die Synthese bestimmter Vitamine. Die typische Gewinnung von GOS umfasst die folgenden Schritte:
1. Konzentrierung einer Milchzuckerlösung (Lactose, Sauermolke, Milchpermeat mit dem Ziel eine Trockenmasse von mindestens 30 Gew.-% zu erreichen;
2. Hocherhitzung/UHT-Behandlung zur Entkeimung bei 85-140 C über 5 bis 300 s;
3. Zugabe einer beta-Galactosidase (z.B. aus *Aspergillus Oryzae oder Bacillus circulans*) und Einstellung der für das Enzym optimalen pH- und Temperaturbedingungen (z.B. pH =4,5, 55 °C);
4. Verweilzeit in der Regel über 30 bis maximal 120 min (abhängig vom Enzym), da ansonsten eine Rückspaltung erfolgt;
5. Inaktivierung des Enzyms beispielsweise durch Hochtemperaturpasteurisierung (90 °C, 10 min) oder pH-Shift;
6. Aufreinigung, gegebenenfalls Konzentrierung, Sprühtrocknung.

### Alpha-Galactooligosaccharide (α-GOS) (Komponente (b2)

Alpha-Galactooligosaccharide können durch Transgalactosylierung von alpha Galactosiden (zum Beispiel Melibiose) durch das Enzym alpha-Galactosidase hergestellt werden. Alpha GOS können bis zu 6 Galactosemonomere enthalten und weisen ein endständiges Glucose Molekül auf. Diese sind Alpha-(1,6) miteinander verknüpft. Sie haben ähnliche prebiotische Eigenschaften wie beta Galactooligosaccharide.

### Fructooligosaccharide (FOS) (Komponente b3)

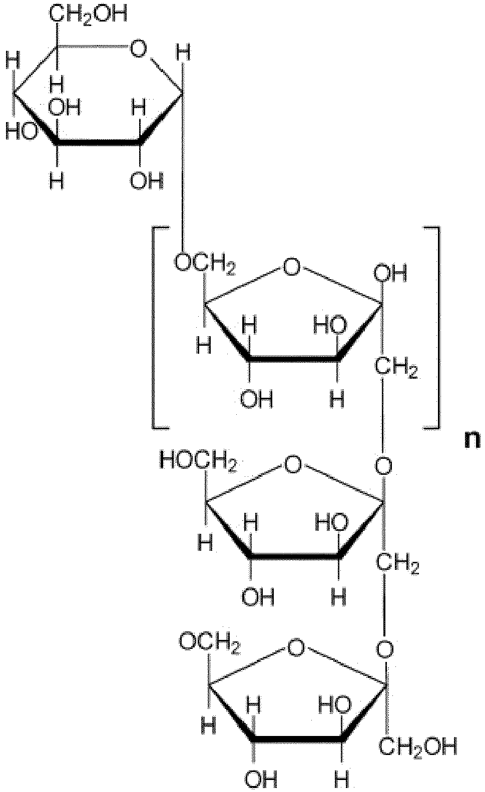

FOS sind Mehrfachzucker die bis zu 10 Monomere enthalten können. Durch Transfructosylierung mittels einer beta-Fructosidase können aus Saccharose Fructooligosaccharide gewonnen werden. Diese weisen neben einer beta 1-2 Verknüpfung auch andere Verknüpfungsmöglichkeiten der monomere Zucker auf. FOS können ebenfalls durch die enzymatische oder säurekatalysierte Hydrolyse von hoch polymerisiertem Inulin hergestellt werden. Da diese glykosidischen Bindungen durch Speichel- und Verdauungsenzyme nicht aufgespalten werden können ergibt sich ein prebiotischer Effekt.

### Mannanoligosaccharide (MOS) (Komponente b4)

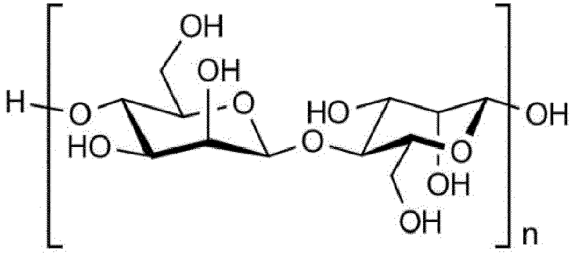

MOS sind Mannanoligosaccharide und kommen natürlicherweise als Bestandteile von Zellwänden in Hefen vor. Sie kommen auch als Mannose-Protein Komplexe vor. MOS Produkte sind aufgereinigte Hefezellwandprodukte unterschiedlicher Hefestämme.

Sowohl aus ernährungsphysiologischen Gründen als auch im Hinblick auf eine optimale Maskierung der bitteren Geschmacksnote der Proteine, sind Oligosaccharide mit einem hohen DP ("degree of polymerisation") bevorzugt, insbesondere mit einem DP im Bereich von etwa 10 bis etwa 80, vorzugsweise etwa 30 bis etwa 70 und insbesondere etwa bis etwa 60. Solche Oligosaccharide werden beispielsweise erhalten, wenn man die Transgalactosilierung zweistufig mit zwei unterschiedlichen Enzymen durchführt.

### ZUBEREITUNGEN

Die erfindungsgemäßen prebiotischen Zubereitungen können weiterhin Hilfs- und Zusatzstoffe enthalten, die ausgewählt sind aus der Gruppe, die gebildet wird von tierischen und/oder pflanzlichen Fetten, Stärkeprodukten, Ballaststoffen, Verdickungsmittel, Aromastoffen, weiteren Prebiotika, Probiotika, Genusssäuren, Salzen und Farbstoffen sowie deren Gemischen.

### Tierische und/oder pflanzliche Fette

Fette (Komponente c1) stelle Lipide dar, die im Wesentlichen gesättigte Fettsäuren enthalten und daher bei Umgebungstemperatur fest vorliegen. Zu den festen tierischen Fetten gehören Rindertalg, Schweineschmalz und Milchfett. Beispiele für Pflanzenfette sind Kokosfett, Palmfett, Palmkernfett und Sheabutter.

### Stärkeprodukte

Im Sinne der vorliegenden Erfindungen umfasst der Begriff Stärkeprodukte, (Komponente c2) bilden natürliche Stärken ebenso wie chemisch oder enzymatisch modifizierte Stärken, sofern diese für die menschliche Ernährung zugelassen sind sowie Dextrine.

**Kartoffelstärke.** Kartoffeln enthalten etwa 75 % Wasser, 21 % Stärke und 4 % andere Substanzen. Zur Herstellung von Kartoffelstärke werden sie traditionell auf schnell rotierenden, mit Sägezähnen besetzten Zylindern unter Zufluss von Wasser möglichst fein zerrieben. Daraufhin wäscht man den Brei, in welchem die Zellen möglichst vollständig zerrissen, die Stärkekörner also bloßgelegt sein sollten, aus einem Metallsieb, auf dem Bürsten langsam rotieren, mit Wasser aus. Bei größeren Betrieben benutzt man kontinuierlich wirkende Apparate, bei denen der Brei durch eine Kette allmählich über ein langes, geneigt liegendes Sieb transportiert und dabei ausgewaschen und das auf den schon fast erschöpften Brei fließende Wasser, welches also nur sehr wenig Stärkemehl aufnimmt, auch noch auf frischen Brei geleitet wird. Der ausgewaschene Brei (Pülpe) enthält 80-95 % Wasser, in der Trockensubstanz aber noch etwa 60 % Stärke und dient als Viehfutter, auch zur Stärkezucker-, Branntwein- und Papierherstellung; das Waschwasser hat man zum Berieseln der Wiesen benutzt, doch gelang es auch, die stickstoffhaltigen Bestandteile des Kartoffelfruchtwassers als Viehfutter zu verwerten. Da die Pülpe noch sehr viel Stärke enthält, zerreibt man sie zwischen Walzen, um alle Zellen zu öffnen, und wäscht sie noch einmal aus. Nach einer anderen Methode schneidet man die Kartoffeln in Scheiben, befreit sie durch Mazeration in Wasser von ihrem Saft und schichtet sie mit Reisigholz oder Horden zu Haufen, in welchen sie bei einer Temperatur von 30-40 °C in etwa acht Tagen vollständig verrotten und in eine lockere, breiartige Masse verwandelt werden, aus welcher die Stärke leicht ausgewaschen werden kann. Das von den Sieben abfließende Wasser enthält die Saftbestandteile der Kartoffeln gelöst und Stärke und feine Fasern, die durch das Sieb gegangen sind, suspendiert. Man rührt dieses Wasser in Bottichen auf, lässt es kurze Zeit stehen, damit Sand und kleine Steinchen zu Boden fallen können, lässt es dann durch ein feines Sieb fließen, um gröbere Fasern zurückzuhalten, und bringt es dann in einen Bottich, in welchem sich die Stärke und auf ihr die Faser ablagert. Die obere Schicht des Bodensatzes wird deshalb nach dem Ablassen des Wassers entfernt und als Schlammstärke direkt verwertet oder weiter gereinigt, indem man sie auf einem Schüttelsieb aus feiner Seidengaze, durch deren Maschen die Stärke, aber nicht die Fasern hindurchgehen, mit viel Wasser auswäscht. Die Hauptmasse der Stärke wird im Bottich wiederholt mit reinem Wasser angerührt und nach jedesmaligem Absetzen von der oberen unreinen Stärke befreit. Man kann auch die rohe Stärke mit Wasser durch eine sehr schwach geneigte Rinne fließen lassen, in deren oberem Teil sich die schwere reine Stärke ablagert, während die leichteren Fasern von dem Wasser weiter fortgeführt werden.

Oft benutzt man auch Zentrifugalmaschinen, in welchen sich die schwere Stärke zunächst an der senkrechten Wand der schnell rotierenden Siebtrommel ablagert, während die leichte Faser noch im Wasser suspendiert bleibt. Das Wasser aber entweicht durch die Siebwand, und man kann schließlich die Stärke aus der Zentrifugalmaschine in festen Blöcken herausheben, deren innere Schicht die Faser bildet. Die feuchte (grüne) Stärke, welche etwa 33-45 % Wasser enthält, wird ohne weiteres zu Traubenzucker verarbeitet, für alle anderen Zwecke aber auf Filterpressen oder auf Platten aus gebranntem Gips, die begierig Wasser einsaugen, auch unter Anwendung der Luftpumpe entwässert und bei einer Temperatur unter 60 °C getrocknet. Man bringt sie in Brocken oder, zwischen Walzen zerdrückt und gesiebt, als Mehl in den Handel. Bisweilen wird die feuchte Stärke mit etwas Kleister angeknetet und durch eine durchlöcherte eiserne Platte getrieben, worauf man die erhaltenen Stängel auf Horden trocknet. Um einen gelblichen Ton der Stärke zu verdecken, setzt man ihr vor dem letzten Waschen etwas Ultramarin zu.

**Weizenstärke.** Weizenstärke wird aus weißem, dünnhülsigem, mehligem Weizen hergestellt. Dieser enthält etwa 58-64 % Stärke, außerdem etwa 10 % Kleber und 3-4 % Zellstoff, welcher hauptsächlich die Hülsen des Korns bildet. Die Eigenschaften des Klebers bedingen die Abweichungen der Weizenstärkefabrikation von der Gewinnung der Stärke aus Kartoffeln. Nach dem traditionellen Halleschen oder Sauerverfahren weicht man den Weizen in Wasser, zerquetscht ihn zwischen Walzen und überlässt ihn, mit Wasser übergossen, der Gärung, die durch Sauerwasser aus einem früheren Prozess eingeleitet wird und Essig- und Milchsäure liefert, in welcher sich der Kleber löst oder wenigstens seine zähe Beschaffenheit so weit verliert, dass man nach 10-20 Tagen in einer siebartig durchlöcherten Waschtrommel die Stärke abscheiden kann. Das aus der Trommel abfließende Wasser setzt in einem Bottich zunächst Stärke, dann eine innige Mischung von Stärke mit Kleber und Hülsenteilchen (Schlichte, Schlammstärke), zuletzt eine schlammige, vorwiegend aus Kleber bestehende Masse ab. Diese Rohstärke wird ähnlich wie die Kartoffelstärke gereinigt und dann getrocknet, wobei sie zu Pulver zerfällt oder, wenn sie noch geringe Mengen Kleber enthält, die so genannte Strahlenstärke liefert, die von den Normalverbrauchern irrtümlich für besonders reingehalten wird.

Nach dem traditionellen Elsässer Verfahren wird der gequollene Weizen durch aufrechte Mühlsteine unter starkem Wasserzufluss zerquetscht und sofort ausgewaschen. Das abfließende Wasser enthält neben Stärke viel Kleber und Hülsenteilchen und wird entweder der Gärung überlassen und dann wie beim vorigen Verfahren weiterverarbeitet, oder direkt in Zentrifugalmaschinen gebracht, wo viel Kleber abgeschieden und eine Rohstärke erhalten wird, die man durch Gärung etc. weiter reinigt. Die bei diesem Verfahren erhaltenen Rückstände besitzen beträchtlich höheren landwirtschaftlichen Wert als die bei dem Halleschen Verfahren entstehenden. Will man aber den Kleber noch vorteilhafter verwerten, so macht man aus Weizenmehl einen festen, zähen Teig und bearbeitet diesen nach etwa einer Stunde in Stücken von 1 kg in einem rinnenförmigen Trog unter Zufluss von Wasser mit einer leicht kannelierten Walze. Hierbei wird die Stärke aus dem Kleber ausgewaschen und fließt mit dem Wasser ab, während der Kleber als zähe, fadenziehende Masse zurückbleibt.

**Reisstärke.** Reis enthält 70-75 % Stärke neben 7-9 % unlöslichen, eiweißartigen Stoffen, die aber durch Einweichen des Reises in ganz schwacher Natronlauge größtenteils gelöst werden. Man zerreibt den Reis dann in einer Mühle unter beständigem Zufluss schwacher Lauge, behandelt den Brei in einem Bottich anhaltend mit Lauge und Wasser, lässt kurze Zeit absetzen, damit sich gröbere Teile zu Boden senken, und zieht das Wasser, in welchem reine Stärke suspendiert ist, ab. Aus dem Bodensatz wird die Stärke in einem rotierenden Siebzylinder durch Wasser ausgewaschen, worauf man sie durch Behandeln mit Lauge und Abschlämmen vom Kleber befreit. Die zuerst erhaltene reinere Stärke lässt man absetzen, entfernt die obere unreine Schicht, behandelt das Übrige auf der Zentrifugalmaschine und trocknet die reine Stärke.

**Maisstärke.** Mais weicht man vier- bis fünfmal je 24 Stunden in Wasser von 35 °C, wäscht ihn und lässt ihn dann durch zwei Mahlgänge gehen. Das Mehl fällt in eine mit Wasser gefüllte Kufe mit Flügelrührer und gelangt aus dieser auf Seidengewebe, das nur die grobe Kleie zurückhält. Das mit der Stärke beladene, durch das Gewebe hindurchgegangene Wasser gelangt in Tröge, dann durch zwei feine Gewebe und endlich auf wenig geneigte, 80-100 m lange Schiefertafeln, auf welchen sich die Stärke ablagert. Das abfließende, nur noch Spuren von Stärke enthaltende Wasser lässt man stehen und presst den Absatz zu Kuchen, um ihn als Viehfutter zu verwenden. Besonders bevorzugt ist der Einsatz von Reisstärke oder Maisstärke.

**Dextrine.** Bei Dextrinen bzw. Maltodextrinen handelt es sich um Stärkeabbauprodukte, die von ihrer Molekülgröße her zwischen Oligosacchariden und Stärke liegen. Üblicherweise kommen sie in Form von weißem bzw. hellgelbem Pulver vor. Sie werden hauptsächlich aus Weizen-, Kartoffel-, Tapioka- und Maisstärke durch trockene Erhitzung (> 150 °C) oder unter Säureeinwirkung gewonnen. In der Natur wird Dextrin zum Beispiel von *Bacterium macer*ans erzeugt. Dextrine entstehen auch durch den enzymatischen Abbau von Stärke durch Amylase können. Bevorzugt sind Dextrine mit 5 bis 20 und insbesondere 6 bis 10 Dextrose-Äquivalenten (DE-Einheiten).

### Ballaststoffe

Ballaststoffe (Komponente c3) sind weitgehend unverdauliche Nahrungsbestandteile, meistens Kohlenhydrate, welche meistens in pflanzlichen Lebensmitteln vorkommen und ein wichtiger Bestandteil der menschlichen Ernährung sind. In wiederum einer weiteren bevorzugten Ausführungsform der vorliegenden Erfindung ist der Ballaststoff ausgewählt aus der Gruppe bestehend aus Inulin, Johannisbrotkernmehl, Pektin, Dextrin, Maltodextrin, Cellulose, Lignin und Alginat oder Mischungen davon.

### Emulgatoren

Emulgatoren (Komponente c4) zeichnen sich durch die wichtige Eigenschaft aus, sowohl in Wasser als auch in Fett löslich zu sein. Emulgatoren bestehen meist aus einem fettlöslichen und einem wasserlöslichen Teil. Sie kommen immer dann zum Einsatz, wenn Wasser und Öl zu einer beständigen, homogenen Vermischung gebracht werden sollen. Geeignete Emulgatoren, die in der lebensmittelverarbeitenden Industrie verwendet werden sind ausgewählt aus: Ascorbylpalmitat (E 304) Lezithin (E 322) Phosphorsäure (E 338) Natriumphosphat (E 339) Kaliumphosphat (E 340) Kalziumphosphat (E 341) Magnesiumorthophosphat (E 343) Propylenglykolalginat (E 405) Polyoxyethylen(8)stearat (E 430) Polyoxyethylenstearat (E 431) Ammoniumphosphatide (E 442) Natriumphosphat und Kaliumphosphat (E 450) Natriumsalze der Speisefettsäuren (E 470 a) Mono- und Diglyceride von Speisefettsäuren (E 471) Essigsäuremonoglyceride (E 472 a) Milchsäuremonoglyceride (E 472 b) Zitronensäuremonoglyceride (E 472 c) Weinsäuremonoglyceride (E 472 d) Diacetylweinsäuremonoglyceride (E 472 e) Zuckerester von Speisefettsäuren (E 473) Zuckerglyceride (E 474) Polyglyceride von Speisefettsäuren (E 475) Polyglycerin-Polyricinoleat (E 476) Propylenglykolester von Speisefettsäuren (E 477) Natriumstearoyllaktylat (E 481) Calciumstearoyl-2-lactylat (E 482) Stearyltartrat (E 483) Sorbitanmonostearat (E 491) Stearinsäure (E 570).

### Verdickungsmittel

Verdickungsmittel (Komponente c5) sind Stoffe, die in erster Linie in der Lage sind, Wasser zu binden. Durch Entzug von ungebundenem Wasser kommt es zur Erhöhung der Viskosität. Ab einer für jedes Verdickungsmittel charakteristischen Konzentration treten zu diesem Effekt auch noch Netzwerkeffekte auf, die zu einer meist überproportionalen Erhöhung der Viskosität führen. Man spricht in diesem Fall davon, dass Moleküle miteinander 'kommunizieren', d.h. verschlaufen. Bei den meisten Verdickungsmitteln handelt es sich um lineare oder verzweigte Makromoleküle (z. B. Polysaccharide oder Proteine), die durch intermolekulare Wechselwirkungen, wie Wasserstoffbrücken, hydrophobe Wechselwirkungen oder lonenbeziehungen miteinander interagieren können. Extremfälle von Dickungsmitteln sind Schichtsilikate (Bentonite, Hectorite) oder hydratisierte SiOz-Partikel, die als Teilchen dispergiert vorliegen und in ihrer festkörperartigen Struktur Wasser binden können bzw. aufgrund der beschriebenen Wechselwirkungen miteinander interagieren können.

Als Verdickungsmittel kommt klassisch vor allem Gelatine in Betracht. Da tierisch basierte Produkte inzwischen vielfach unerwünscht sind, können alternativ pflanzliche Produkte verwendet werden, beispielsweise:
E 400 - Alginsäure
E 401 - Natriumalginat
E 402 - Kaliumalginat
E403 - Ammoniumalginat
E404 - Calciumalginat
E 405 - Propylenglycolalginat
E406 - Agar Agar
E407 - Carrgeen, Furcelleran
E407 - Johannisbrotkernmehl
E 412 - Guarkernmehl
E413 - Traganth
E 414 - Gummi arabicum
E415 - Xanthan
E 416 - Karaya (Indischer Traganth)
E 417 - Tarakernmehl (Peruanisches Johannisbrotkernmehl)
E 418 - Gellan
E 440 - Pektin, Opekta
E 440ii - Amidiertes Pektin
E 460 - Mikrokristalline Cellulose, Cellulosepulver
E 461 - Methylcellulose
E 462 - Ethylcellulose
E 463 - Hydroxypropylcellulose
E 465 - Methylethylcellulose
E 466 - Carboxymethylcellulose, Natriumcarboxymethylcellulose

### Aromastoffe

Die erfindungsgemäßen Zubereitungen können einen oder mehrere Aromastoffe (Komponente c6) enthalten. Typische Beispiele umfassen: Acetophenon, Allylcapronat, alphalonon, beta-Ionon, Anisaldehyd, Anisylacetat, Anisylformiat, Benzaldehyd, Benzothiazol, Benzylacetat, Benzylalkohol, Benzylbenzoat, beta-Ionon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion^{®}), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methyl-pyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

Die Aromastoffe können auch in fester oder pastöser Form, beispielsweise als getrocknete Würzmischungen oder kleingeschnittene Kräuter zugegeben werden.

### Probiotische Mikroorganismen

Probiotische Mikroorganismen, auch als "Probiotics" bezeichnet (Komponente c7) stellen lebende Mikroorganismen dar, die für den Wirt nützliche Eigenschaften besitzen. Gemäß der Definition der FAO/WHO handelt es sich um "lebende Mikroorganismen, die bei angemessener Dosierung dem Wirt einen Gesundheitsvorteil vermitteln". Milchsäurebakterien (LAB) und Bifidobakterien stellen die bekanntesten Probiotics dar; es können aber auch verschiedene Hefen und Bazillen Verwendung finden. Probiotics werden üblicherweise als Bestandteil von fermentierten Nahrungsmitteln aufgenommen, denen man spezielle Lebendkulturen zugesetzt hat, wie z.B. Joghurt, Sojajoghurt oder andere probiotische Nahrungsmittel. Darüber hinaus sind auch Tabletten, Kapseln, Pulver und Sachets erhältlich, die die Mikroorganismen in gefriergetrockneter Form enthalten. Tabelle A gibt einen Überblick über handelsübliche Probiotics und den zugehörigen Auslobungen zur Gesundheit, die im Sinne der vorliegenden Erfindung als Komponente (b1) eingesetzt werden können.

**Tabelle A**

| Probiotische Mikroorganismen | | | |
|---|---|---|---|
| **Stamm** | **Bezeichnung** | **Hersteller** | **Auslobung** |
| *Bacillus coagulans* GBI-30, 6086 | GanedenBC | Ganeden Biotech | Steigert die Immunantwort bei viraler Infektion |
| *Bifidobacterium animalis* subsp. *lactis* BB-12 | Probio-Tec Bifidobacterium BB-12 | Chr. Hansen | Klinische Studien am Menschen haben gezeigt, dass BB-12 alleine oder in Kombination das gastrotestinale System positiv beeinflusst. |
| *Bifidobacterium infantis* 35624 | Align | Procter & Gamble | In einer vorläufigen Studie wurde gezeigt, dass das Bakterium abdominale Schmerzen verringern kann. |
| *Lactobacillus acidophilus* NCFM | | Danisco | Aus einer Studie geht hervor, dass die Nebeneffekte von antibiotischen Behandlungen verringert werden |
| *Lactobacillus paracasei* St11 (or NCC2461) | | | |
| *Lactobacillus johnsonii* La1 (= Lactobacillus LC1, *Lactobacillus johnsonii* NCC533) | | Nestlé | Verringert Gastritisbeschwerden und vemindert Entzündungen |
| *Lactobacillus plantarum* 299v | GoodBelly/ ProViva/ProbiMag e | Probi | Könnte IBS Symptome verbessern; jedoch noch mehr Studien erforderlich. |
| *Lactobacillus reuteri* American Type Culture CollectionlATTC 55730 (*Lactobacillus reuteri* SD2112) | | BioGaia | Erste Anzeichen für eine Wirksamkeit gegen Gangivitis, Fieber bei Kindern und Verminderung der Krankheitstage bei Erwachsenen. |
| *Lactobacillus reuteri* Protectis (DSM 17938, daughter strain of ATCC 55730) | | | |
| *Saccharomyces boulardii* | DiarSafe and others | Wren Laboratories | Beschränkter Nachweis bei der Behandlung von akuten Durchfallerkrankungen. |
| *Lactobacillus rhamnosus* GR-1 *& Lactobacillus reuteri* RC-14 | Bion Flore Intime/ Jarrow Fem-Dophilus | Chr. Hansen | In einer Studie Nachweis der Wirksamkeit gegen Vaginitis. |
| *Lactobacillus acidophilus* NCFM *& Bifidobacterium bifidum* BB-12 | Florajen3 | American Lifeline, Inc | Erste Hinweise auf Wirksamkeit gegen CDAD |
| *Lactobacillus acidophilus* CL1285 *& Lactobacillus casei* LBC80R | Bio-K+ CL1285 | Bio-K+ International | Hinweise auf Verbesserung bei der Verdauung, speziell im Hinblick auf Lactoseintoleranz. |
| *Lactobacillus plantarum* HEAL 9 *& Lactobacillus paracasei* 8700:2 | Bravo Friscus/ Probi Frisk | Probi | Derzeit laufen Untersuchungen zur Wirksamkeit gegen Erkältungserkrankungen. |

Zwei weitere Formen von Milchsäurebakterien, nämlich *Lactobacillus bulgaricus und Streptococcus thermophilus* eignen sich ebenfalls als Prebiotics. Auch spezielle fermentierte Produkte auf Basis solcher Milchsäurebakterien sind einsetzbar, wie beispielsweise Mixed Pickles, fermentierte Bohnenpaste wie beispielsweise tempeh, miso and doenjang; Kefir; Buttermilch, Kimchi; Pao cai; Sojasoße oder Zha cai.

### Prebiotische Stoffe

In einer weiteren Ausgestaltung der Erfindung können die Zubereitungen zusätzliche prebiotische Stoffe ("Prebiotika") (Komponente c8) enthalten. Prebiotika werden als unverdauliche Nahrungsbestandteile definiert, deren Verabreichung das Wachstum oder die Aktivität einer Reihe nützlicher Bakterien im Dickdarm stimuliert. Die Zugabe von prebiotischen Verbindungen verbessert die Stabilität der Anthocyanine gegenüber Abbauprozessen im Darmtrakt. Im Folgenden werden verschiedene Stoffe, insbesondere Kohlenhydrate, die als Prebiotics im Sinne der Erfindung besonders bevorzugt sind, nämlich insbesondere
- Fructooligosaccharide
- Inuline
- Isomaltooligosaccharide
- Lactilol
- Lactulose
- Pyrodextrine
- Sojaoligosaccharide
- Xylooligosaccharide sowie
- Spezielle Biopolymere

Fructooligosaccharide oder abgekürzt FOS, umfassen insbesondere kurzkettige Vertreter mit 3 bis 5 Kohlenstoffatomen, wie beispielsweise D-Fructose und D-Glucose. FOS, auch als Neozucker bezeichnet, werden kommerziell auf Basis von Saccharose und dem aus Pilzen gewonnenen Enzym Fructosyltransferase hergestellt. FOS unterstützen insbesondere das Wachstum von Bifidobakterien im Darm und werden vor allem in den USA zusammen mit probiotischen Bakterien in verschiedenen funktionalisierten Lebensmitteln vermarktet.

Inuline zählen zu einer Gruppe von natürlich vorkommenden Fructose enthaltenden Oligosacchariden. Sie gehören zu einer Klasse von Kohlenhydraten, die als Fructane bezeichnet werden. Ihre Gewinnung erfolgt aus den Wurzeln der Chicoree-Pflanze (Cichorium intybus) oder so genannten Jerusalem-Artischocken. Inuline bestehen überwiegend aus Fructoseeinheiten und weisen typisch eine Glucose Einheit als Endgruppe auf. Die Fructoseeinheiten sind dabei miteinander über eine beta-(2-1)glykosidische Bindung verknüpft. Der mittlere Polymerisationsgrad von Inulinen, die als Prebiotics im Nahrungsmittelbereich Anwendung finden, liegt bei 10 bis 12. Inuline stimulieren ebenfalls das Wachstum von Bifidobakterien im Dickdarm.

Isomaltooligosaccharide stellen eine Mischung von alpha-D-verknüpften Glucoseoligomeren, einschließlich Isomaltose, Panose, Isomaltotetraose, Isomaltopentaose, Nigerose, Kojibiose, Isopanose und höherer verzweigter Oligosaccharide dar. Isomaltooligosaccharide werden über verschiedene enzymatische Wege hergestellt. Sie stimulieren ebenfalls das Wachstum von Bifidobakterien und Lactobacillen im Dickdarm. Isomaltooligosaccharide werden speziell in Japan als Nahrungsmittelzusatzstoffe in funktionalisierten Lebensmitteln eingesetzt. Inzwischen finden sie auch in den USA Verbreitung.

Lactilol ist das Disaccharid der Lactulose. Seine medizinische Anwendung findet gegen Verstopfung und bei häpatischer Enzephalopathie. In Japan wird Lactilol als prebiotic eingesetzt. Es widersteht dem Abbau im oberen Verdauungstrakt, wird aber durch verschiedene Darmbakterien fermentiert, was zu einem Anstieg der Biomasse an Bifidobakterien und Lactobacillen im Darm führt. Lactilol ist auch unter der chemischen Bezeichnung 4-0-(beta-D-galactopyranosyl)-D-glucitol bekannt. Der medizinische Anwendungsbereich von Lactilol in den USA ist wegen fehlender Studien beschränkt; in Europa wird es vorzugsweise als Süßstoff eingesetzt.

Lactosucrose ist ein Trisaccharid, das sich aus D-Galactose, D-Glucose und D-Fructose aufbaut. Lactosucrose wird durch enzymatischen Transfer des Galactosylrestes in der Lactose auf die Sucrose hergestellt. Es wird weder im Magen noch im oberen Teil des Darmtraktes abgebaut und wird ausschließlich von Bifidobakterien zu Wachstum konsumiert. Unter physiologischen Gesichtspunkten wirkt Lactosucrose als Stimulator für das Wachstum der Darmflora. Lactosucrose ist ebenfalls bekannt als 4G-beta-D-galactosucrose. Es ist in Japan als Nahrungsmittelzusatzstoff und als Bestandteil von funktionalisierten Lebensmitteln weit verbreitet, insbesondere auch als Zusatzstoff für Joghurts. Lactosucrose wird derzeit auch in den USA für einen ähnlichen Anwendungszweck getestet.

Lactulose ist ein halbsynthetisches Disaccharid aus D-Lactose und D-Fructose. Die Zucker sind über eine beta-glykosidische Bindung verknüpft, was sie resistent gegen Hydrolyse durch Verdauungsenzyme macht. Stattdessen wird Lactulose durch eine beschränkte Anzahl von Darmbakterien fermentiert, was zu einem Wachstum insbesondere von Lactobacillen und Bifidobakterien führt. Lactulose stellt in den USA ein verschreibungspflichtiges Medikament gegen Verstopfung und hepatische Enzephalopathie dar. In Japan hingegen wird es als Nahrungsmittelzusatzstoff und Bestandteil von funktionalisierten Lebensmitteln frei verkauft.

Pyrodextrine umfassen eine Mischung von Glucose enthaltenden Oligosacchariden, die bei der Hydrolyse von Stärke gebildet werden. Pyrodextrine fördern die Proliferation von Bifidobakterien im Dickdarm. Auch sie werden im oberen Darmbereich nicht abgebaut.

Sojaoligosaccharide sind spezielle Saccharide, die im Wesentlichen nur in Sojabohnen und darüber noch in anderen Bohnen sowie Erbsen zu finden sind. Die beiden maßgeblichen Vertreter sind das Trisaccharid Raffinose und das Tetrasaccharid Stachyose. Raffinose setzt sich aus jeweils einem Molekül D-Galactose, D-Glucose und D-Fructose zusammen. Stachyose besteht aus zwei Molekülen D-Galactose sowie jeweils einem Molekül D-Glucose und D-Fructose. Sojaoligosacccharide stimulieren das Wachstum von Bifidobakterien im Dickdarm und werden in Japan bereits als Nahrungsmittelzusatzstoffe sowie in funktionalisierten Lebensmitteln eingesetzt. In den USA werden sie für diese Anwendung derzeit getestet.

Xylooligosaccharide enthalten beta-1,4-verknüpfte Xyloseeinheiten. Der Polymerisationsgrad der Xylooligosaccharide liegt zwischen 2 und 4. Sie werden durch enzymatische Hydrolyse des Polysaccharids Xylan erhalten. Sie werden bereits in Japan als Nahrungsmittelzusatzstoffe vermarktet, in den USA befinden sie sich noch in der Testphase.

Geeignete Biopolymere, die ebenfalls als Prebiotics in Betracht kommen, wie beispielsweise Beta-Glucane, zeichnen sich dadurch aus, dass sie auf pflanzlicher Basis hergestellt werden, beispielsweise kommen als Rohstoffquellen Cerealien wie Hafer und Gerste, aber auch Pilze, Hefen und Bakterien in Frage. Außerdem geeignet sind mikrobiell hergestellte Zellwandsuspensionen oder ganze Zellen mit hohem Beta-Glucan Gehalt. Restliche Anteile an Monomeren weisen 1-3 und 1-4 oder 1-3 und 1-6 Verknüpfungen auf, wobei der Gehalt stark variieren kann. Vorzugsweise werden Beta-Glucane auf Basis von Hefen, insbesondere Saccharomyces, speziell Saccharomyces cerevisiae, erhalten. Andere geeignete Biopolymere sind Chitin und Chitinderivate, insbesondere Oligoglucosamin und Chitosan, das ein typisches Hydrokolloid darstellt.

### Genusssäuren

Der Begriff "Genusssäure" (Komponente c9) bezeichnet organische Säuren, Fruchtsäuren oder Phosphorsäuren, welche aufgrund ihres Geschmacks und anderer lebensmitteltechnisch vorteilhaften Eigenschaften als Zusatzstoffe, speziell als Lebensmittelsäuren oder Säureregulatoren in der Lebensmittelproduktion eingesetzt werden, wie beispielsweise:
E 260 - Essigsäure
E 270 - Milchsäure
E 290 - Kohlendioxid
E 296 - Apfelsäure
E 297 - Fumarsäure
E 330 - Citronensäure
E 331 - Natriumcitrat
E 332 - Kaliumcitrat
E 333 - Calciumcitrat
E 334 - Weinsäure
E 335 - Natriumtartrat
E 336 - Kaliumtartrat
E 337 - Natrium-Kaliumtartrat
E 338 - Phosphorsäure
E 353 - Metaweinsäure
E 354 - Calciumtartrat
E 355 - Adipinsäure
E 363 - Bernsteinsäure
E 380 - Triammoniumcitrat
E 513 - Schwefelsäure
E 574 - Gluconsäure
E 575 - Glucono-delta-Lacton

Vorzugsweise, im Rahmen der vorliegenden Erfindung, sind die Genusssäuren ausgewählt aus der Gruppe bestehen aus Milchsäure, Weinsäure, Essigsäure, Apfelsäure, Zitronensäure oder Fumarsäure. Auch wenn ein erfindungsgemäßes Lebensmittelprodukt solche Genusssäuren, die im Rahmen der vorliegenden Erfindung gegebenenfalls (auch) aufgrund ihres Geschmacks eingesetzt werden, enthalten kann, sind die Genusssäuren im Rahmen des vorliegenden Textes nicht dem "Aromastoff" im Sinne des Anspruchswortlauts zuzuordnen (für bevorzugt zu verwendende Aromastoffe s. unten). Solche (weiteren) Aromastoffe sind also stets zusätzlich enthalten.

### Süßstoffe

Die Zubereitungen können des Weiteren Süßstoffe (Komponente c10) enthalten. Da es sich um kalorienarme oder wenigstens kalorienreduzierte Lebensmittel handelt, ist es zwar möglich, zum Süßen konventionellen Zucker einzusetzen, es ist jedoch weniger bevorzugt. Stattdessen werden vorzugsweise pflanzliche Alternativen eingesetzt.

Als Süßstoffe oder süß schmeckende Zusatzstoffe kommen zunächst Kohlenhydrate und speziell Zucker in Frage, wie etwa Sucrose/Saccharose, Trehalose, Lactose, Maltose, Melizitose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glycerinaldehyd, oder Maltodextrin. Ebenfalls geeignet sind pflanzliche Zubereitungen, die diese Stoffe enthalten, beispielsweise auf Basis von Zuckerrüben (Beta vulgaris ssp., Zuckerfraktionen, Zuckersirup, Melasse), Zuckerrohr (Saccharum officinarum ssp., Melasse, Zuckerrohrsirup), Ahornsirup (Acer ssp.) oder Agaven (Agavendicksaft).

In Betracht kommen auch
- synthetische, d.h. in der Regel enzymatisch hergestellte Stärke oder Zuckerhydrolysate (Invertzucker, Fructosesirup);
- Fruchtkonzentrate (z.B. auf Basis von Äpfeln oder Birnen);
- Zuckeralkohole (z.B. Erythritol, Threitol, Arabitol, Ribotol, Xylitol, Sorbitol, Mannitol, Dulcitol, Lactitol);
- Proteine (z.B. Miraculin, Monellin, Thaumatin, Curculin, Brazzein);
- Süßstoffe (z.B. Magap, Natriumcyclamat, Acesulfam K, Neohesperidin Dihydrochalcon, Saccharin Natriumsalz, Aspartam, Superaspartam, Neotam, Alitam, Sucralose, Stevioside, Rebaudioside, insbesondere A und D, Lugduname, Carrelame, Sucrononate, Sucrooctate, Monatin, Phenylodulcin);
- Süß schmeckende Aminosäuren (z.B. Glycin, D-Leucin, D-Threonin, D-Asparagin, D-Phenylalanin, D-tryptophn, L-Prolin);
- Weitere süß schmeckende niedermolekulare Substanzen, wie z.B. Hernandulcin, Dihydrochalconglykoside, Glycyrrhizin, Glycerrhetinsäure, ihre Derivate und Salze, Extrakte von Lakritz (Glycyrrhizza glabra ssp.), Lippia dulcis Extrakte, Momordica ssp. Extrakte oder
- Einzelsubstanzen wie z.B. Momordica grosvenori [Luo Han Guo] und die daraus gewonnenen Mogroside, Hydrangea dulcis oder Stevia ssp. (z.B. Stevia rebaudiana) sowie Rubus Extrakte.

### Antioxidantien und Vitamine

In einer weiteren Ausführungsform der vorliegenden Erfindung können die Zubereitungen Antioxidantien und/oder Vitamine (Komponente c11) enthalten.

In der Lebensmittelindustrie werden sowohl natürliche als auch künstliche Antioxidationsmittel verwendet. Natürliche und künstliche Antioxidantien unterscheiden sich in erster Linie dadurch, dass erstere natürlich in der Nahrung vorkommen und letztere künstlich hergestellt werden. So werden natürliche Antioxidationsmittel, so sie als Lebensmittelzusatzstoff eingesetzt werden sollen, beispielsweise aus Pflanzenölen gewonnen. Vitamin E - auch als Tocopherol bekannt-wird beispielsweise häufig aus Sojaöl hergestellt. Synthetische Antioxidantien wie das Propylgallat, das Octylgallat und das Dodecylgallat werden dagegen durch chemische Synthese gewonnen. Die Gallate können bei empfindlichen Personen Allergien auslösen. Weitere einsetzbare Antioxidantien in Zusammensetzungen der vorliegenden Erfindung sind: Schwefeldioxid, E 220 Sulfite Natriumsulfit, E 221 Natriumhydrogensulfit, E 222 Natriumdisulfit, E 223 Kaliumdisulfit, E 224 Kalziumsulfit, E 226 Kalziumhydrogensulfit, E 227 Kaliumhydrogensulfit, E 228 Milchsäure, E 270 Ascorbinsäure, E 300 Natrium-L-Ascorbat, E 301 Calcium-L-Ascorbat, E 302 Ascorbinsäureester, E 304 Tocopherol, E 306 Alpha-Tocopherol, E 307 Gamma-Tocopherol, E 308 Delta-Tocopherol, E 309 Propylgallat, E 310 Octygallat, E 311 Dodecylgallat, E 312 Isoascorbinsäure, E 315 Natriumisoascorbat, E 316 tertiär-Butylhydrochinon (TBHQ), E 319 Butylhydroxianisol, E 320 Butylhydroxitoluol, E 321 Lecithin, E 322 Citronensäure, E 330 Salze der Zitronensäure (E 331 & E 332) Natriumzitrat, E 331 Kaliumzitrat, E 332 Calcium-Dinatrium-EDTA, E 385 Diphosphate, E 450 Dinatriumdiphosphat, E 450a Trinatriumdiphosphat, E 450b Tetranatriumdiphosphat, E 450c Dikaliumdiphosphat, E 450d Trekaliumdiphosphat, E 450e Dikalziumdiphosphat, E 450f Kalziumdihydrogendiphosphst, E 450g Triphosphate, E 451 Pentanatriumtriphosphat, E 451a Pentakaliumtriphosphat, E 451b Polyphosphat, E 452 Natriumpolyphosphat, E 452a Kaliumpolyphosphat, E 452b Natriumkalziumpolyphosphat, E 452c Kalziumpolyphosphat, E 452d Zinn-II-Chlorid, E 512.

Vitamine verfügen über unterschiedlichste biochemische Wirkungsweisen. Einige wirken ähnlich wie Hormone und regulieren den Mineralmetabolismus (z.B. Vitamin D), oder wirken auf das Wachstum von Zellen und Gewebe sowie die Zelldifferenzierung (z.B. einige Formen des Vitamin A). Andere stellen Antioxidantien dar (z.B. Vitamin E und unter bestimmten Umständen auch Vitamin C). Die größte Zahl von Vitaminen (z.B. die B-Vitamine) stellen Vorstufen für enzymatische Co-Faktoren dar, die Enzyme dabei unterstützen, bestimmte Prozesse im Metabolismus zu katalysieren. In diesem Zusammenhang können Vitamin mitunter eng an die Enzyme gebunden sein, beispielsweise als Teil der prostetischen Gruppe: ein Beispiel hierfür ist Biotin, das ein Teil des Enzyms ist, welches für den Aufbau von Fettsäuren verantwortlich ist. Vitamine können andererseits auch weniger stark gebunden sein und dann als Co-Katalysatoren wirken, beispielsweise als Gruppen, die sich leicht abspalten lassen und chemische Gruppen oder Elektronen zwischen den Molekülen transportieren. So transportiert beispielsweise Folsäure Methyl-, Formyl- und Methylengruppen in die Zelle. Obwohl ihre Unterstützung in Enzym-Substrat-Reaktionen wohl bekannt ist, sind auch ihre übrigen Eigenschaften für den Körper von großer Bedeutung.

Im Rahmen der vorliegenden Erfindung kommen als Vitamine Stoffe in Betracht, die ausgewählt sind aus der Gruppe bestehend aus
- Vitamin A (Retinol, Retinal, Betakarotin),
- Vitamin B₁ (Thiamin),
- Vitamin B₂ (Rioflavin),
- Vitamin B₃ (Niacin, Niacinamid),
- Vitamin B₅ (Panthothensäure),
- Vitamin B₆ (Pyridoxin, Pyridoxamin, Paridoxal),
- Vitamin B₇ (Biotin),
- Vitamin B₉ (Folsäure, Folinsäure),
- Vitamin B₁₂ (Cyanobalamin, Hydroxycobalmin, Methylcobalmin),
- Vitamin C (Ascorbinsäure),
- Vitamin D (Cholecalciferol),
- Vitamin E (Tocopherole, Tocotrienole) und
- Vitamin K (Phyllolchinon, Menachinon).
Die bevorzugten Vitamine sind neben der Ascorbinsäure die Gruppe der Tocopherole.

### Salze

Als Salze (Komponente c13) kommen Natriumchlorid, Magnesiumchlorid und Kaliumchlorid in Frage.

### Lebensmittelfarbstoffe

Lebensmittelfarbstoffe oder kurz Farbstoffe (Komponente c14) sind Lebensmittelzusatzstoffe zum Färben von Lebensmittel. Farbstoffe werden in die Gruppen der natürlichen Farbstoffe und synthetischen Farbstoffe unterteilt. Die naturidentischen Farbstoffe sind ebenfalls synthetischen Ursprungs. Die naturidentischen Farbstoffe sind synthetische Nachbildungen von in der Natur vorkommenden, färbenden Substanzen. Geeignete Farbstoffe für den Einsatz in der vorliegenden Zusammensetzung sind ausgewählt aus: Kurkumin, E 100 Riboflavin, Lactoflavin, Laktoflavin, Vitamin B2, E 101 Tartrazin, E 102 Chinolingelb, E 104 Gelborange S, Gelborange RGL, E 110 Cochenille, Karminsäure, echtes Karmin, E 120 Azorubin, Carmoisin, E 122 Amaranth, E 123 Cochenillerot A, Ponceau 4 R, Victoriascharlach 4 R, E 124 Erythrosin, E 127 Allurarot AC, E 129 Patentblau V, E 131 Indigotin, Indigo-Karmin, E 132 Brillantblau FCF, Patentblau AE, Amidoblau AE, E 133 Chlorophylle, Chlorophylline, E 140 Kupferkomplexe der Chlorophylle, Kupfer-Chlorophyllin-Komple, E 141 Brillantsäuregrün, Grün S, E 142 Zuckerkulör, Zuckercouleur, E 150 a Sulfitlaugen-Zuckerkulör, E 150 b Ammoniak-Zuckerkulör, E 150 c Ammoniumsulfit-Zuckerkulör, E 150 d Brillantschwarz FCF, Brillantschwarz PN, Schwarz PN, E 151 Pflanzenkohle, E 153 Braun FK, E 154 Braun HAT, E 155 Carotin, Karotin, E 160 a Annatto, Bixin, Norbixin, E 160 b Capsanthin, Capsorubin, E 160 c Lycopin, E 160 d Beta-apo-8'-Carotinal, Apocarotinal, Beta-Apocarotinal, E 160 e Beta-apo-8'-Carotinsäure-Ethylester (C30), Apocarotinester, Beta-Carotinsäureester, E 160 f Lutein, Xanthophyll, E 161 b Canthaxanthin, E 161 g Betanin, Betenrot, E 162 Anthocyane, E 163 Calciumcarbonat, E 170 Titandioxid, E 171 Eisenoxide, Eisenhydroxide, E 172 Aluminium, E 173 Silber, E 174 Gold, E 175 Litholrubin BK, Rubinpigment BK, E 180.

### Bevorzugte Mischungen

In einer bevorzugten Ausführungsform können die Zubereitungen jeweils bezogen auf das Gesamtgewicht folgende Zusammensetzung aufweisen:
(a) etwa 10 bis etwa 95, vorzugsweise etwa 50 bis etwa 80 Gew.-% Milchproteine und/oder pflanzliche Proteine,
(b) etwa 5 bis etwa 20, vorzugsweise etwa 10 bis etwa 15 Gew.-% Oligosaccharide;
(c1) 0 bis etwa 20, vorzugsweise etwa 1 bis etwa 10 Gew.-% tierische und/oder pflanzliche Fette
(c2) 0 bis etwa 5 Gew.-%, vorzugsweise etwa 1 bis etwa 2 Gew.-% Stärkeprodukte;
(c3) 0 bis etwa 5 Gew.-%, vorzugsweise etwa 1 bis etwa 2 Gew.-% Ballaststoffe;
(c4) 0 bis etwa 5 Gew.-%, vorzugsweise etwa 1 bis 2 etwa Gew.-% Emulgatoren;
(c5) 0 bis etwa 2 Gew.-%, vorzugsweise etwa 0,5 bis etwa 1 Gew.-% Verdickungsmittel;
(c6) 0 bis etwa 2 Gew.-%, vorzugsweise etwa 0,5 bis etwa 1 Gew.-% Aromastoffe;
(c7) 0 bis etwa 1 Gew.-%, vorzugsweise etwa 0,1 bis etwa 0,5 Gew.-% probiotische Mikroorganismen;
(c8) 0 bis etwa 1 Gew.-%, vorzugsweise etwa 0,1 bis etwa 0,5 Gew.-% probiotische Stoffe;
(c9) 0 bis etwa 1 Gew.-%, vorzugsweise etwa 0,1 bis etwa 0,5 Gew.-% Genusssäuren,
(c10) 0 bis etwa 1 Gew.-%, vorzugsweise etwa 0,1 bis etwa 0,5 Gew.-% Süßstoffe;
(c11) 0 bis etwa 1 Gew.-%, vorzugsweise etwa 0,1 bis 0,5 Gew.-% Antioxidantien und/oder Vitamine;
(c12) 0 bis etwa 1 Gew.-% Lab;
(c15) 0 bis etwa 1 Gew.-% Salze;
(c14) 0 bis etwa 0,5 Gew.-% Farbstoffe;
mit der Maßgabe, dass sich die Mengenangaben gegebenenfalls mit Wasser zu 100 Gew.-% ergänzen. Nachfolgend werden die weiteren Hilfs- und Zusatzstoffe beispielhaft erläutert. Der guten Ordnung halber sei darauf hingewiesen, dass Zubereitungen, die sich auf diese Weise nicht zu 100 Gew.-% ergänzen nicht Gegenstand der Erfindung sind und es dem Fachmann auf der Grundlage der vorliegenden Beschreibung jederzeit möglich ist, Zubereitungen auszuwählen, deren Komponenten sich zu 100 Gew.-% ergänzen und die technische Lehre erfüllen.

Die Zubereitungen können als wässrige Lösungen oder Dispersionen vorliegen, vorzugsweise handelt es sich jedoch um Pulver, speziell trockene Pulver, d.h. dass sie eine Restfeuchte von weniger als 5 Gew.-% aufweisen.

Im Zusammenhang mit der Darreichungsform der Zubereitungen wird auch ein Verfahren zur Herstellung der oben beschriebenen prebiotischen Zubereitungen nach beansprucht, das folgende Schritte umfasst oder daraus besteht:
(i) Bereitstellen einer wässrigen Lösung der Proteinkomponente;
(ii) Bereitstellen einer wässrigen Lösung der Oligosaccharid-Komponente;
(iii) Vermischen der beiden Komponenten; gegebenenfalls
(iv) Entwässern der Mischung; sowie gegebenenfalls
(v) Zumischung der weiteren Zusatzstoffe.

Neben der Gefriertrocknung - die in der Regel zu teuer sein wird - ist die Sprühtrocknung die bevorzugte Entwässerungsmethode. Dabei werden die aufkonzentrierten und vorgewärmten Lösungen oder Dispersionen enthaltend die Komponenten (a) und (b) bei Temperaturen von etwa 80 bis 95 °C über einen Turm versprüht.

### GEWERBLICHE ANWENDBARKEIT

Ein weiterer Gegenstand der Erfindung betrifft die Verwendung der Zubereitungen zum einen als Nahrungsergänzungsmittel und zum anderen für die Tierernährung.

Ein weiterer Gegenstand der Erfindung betrifft schließlich die Verwendung der genannten Oligosaccharide zur Maskierung des bitteren Geschmacks von pflanzlichen Proteinen. Dabei reicht es bereits aus, den Proteinen Oligosaccharide in Mengen von etwa 1 bis etwa 5 Gew.-% zuzusetzen.

### BEISPIELE

### Herstellbeispiel 1

### Herstellung von β-GOS mit hohem DP ausgehend von Lactoselösung

1.000 kg einer 30 Gew.-%igen Lactoselösung wurde in einem Röhrenwärmeaustauscher über 120 Sekunden auf 98 C erhitzt und dabei entkeimt. Die entkeimte Lösung wurde auf 55 C abgekühlt, in einen ersten Fermenter überführt, mit Hilfe von Milchsäure auf einen pH-Wert von 6,5 eingestellt, mit beta-Galactosidase *aus Bacillus circulans* im Gewichtsverhältnis Enzym:Substrat von 1:500 versetzt und gerührt. Der Fortschritt der Transgalactosilierung wurde durch Probennahme verfolgt. Nach etwa 90 Minuten war die maximale GOS-Konzentration erreicht. Der pH-Wert wurde durch Zugabe von 30 Gew.-%iger Natronlauge innerhalb von wenigen Minuten auf 10 erhöht, wodurch die Aktivität des Enzyms schlagartig um 80 % verringert wurde. Der Reaktionsansatz wurde zu einer Ultrafiltrationseinheit geleitet, die mit einer Spiralwickelmembran mit einer Porengröße von 10 kDa versehen war. Das inaktivierte Enzymmaterial wurde mit dem Retentat R1 in den Fermenter zurückgeführt; um Verlust auszugleichen wurden bezogen auf die Ausgangsmenge 5 Gew.-% frisches Enzym zugefügt.

Das Permeat P1 wurde in einen zweiten Fermenter überführt, mit beta-Galactosidase aus *Aspergillus oryzae* im Gewichtsverhältnis Enzym:Substrat von 1:500 versetzt und nach Einstellung des pH-Wertes auf das Enzymoptimum gerührt. Der Fortschritt der Transgalactosilierung wurde wieder durch Probennahme verfolgt. Nach etwa 90 Minuten war die maximale GOS-Konzentration erreicht. Der pH-Wert wurde durch Zugabe von 30 Gew.-%iger Natronlauge innerhalb von wenigen Minuten auf 10,0 eingestellt, wodurch die Aktivität des Enzyms schlagartig um 80 % verringert wurde.

Der Reaktionsansatz wurde zu einer zweiten Ultrafiltrationseinheit geleitet, die ebenfalls mit einer Spiralwickelmembran mit einer Porengröße von 10 kDa versehen war. Das inaktivierte Enzymmaterial wurde mit dem Retentat R2 in den Fermenter zurückgeführt; um Verlust auszugleichen wurden bezogen auf die Ausgangsmenge 5 Gew.-% frisches Enzym zugefügt.

Das Permeat P2 wurde zu einer Nanofiltrationseinheit geleitet, die mit einer Keramikmembran mit einer Porenweite von 1.000 Da versehen war. Mit dem Permeat P3 wurden die noch im Produkt enthaltenen Monosaccharide abgetrennt, während das Retentat R3 einer Umkehrosmoseeinheit zugeleitet wurde, die mit einem Konzentrierungsfaktor von 1:2 arbeitete. Das dabei anfallende Permeat P3 (also das Konzentrierungswasser) wurde in das Verfahren zurückgeleitet, das Retentat R3 (also das GOS-Konzentrat) wurde im Plattenwärmeaustauscher 30 Sekunden auf etwa 85 C erhitzt und über einen Turm versprüht. Es wurde ein weißes Pulver mit einem GOS-Gehalt von mehr als 75 Gew.-% erhalten, welches noch eine Restfeuchte von 1 Gew.-% aufwies. Der mittlere DP der GOS betrug 3-4.

### Herstellbeispiel 2

### Herstellung von GOS nach dem neutralen Verfahren ausgehend von Saccharoselösung

1000 kg einer 30 Gew.-%igen Sachcharoselösung wurde in einem Röhrenwärmeaustauscher über 120 Sekunden auf 98 °C erhitzt und dabei entkeimt. Die entkeimte Lösung wurde auf 55 °C abgekühlt, in einen Fermenter überführt, mit Hilfe von Milchsäure auf einen pH-Wert von 4,5 eingestellt, mit alpha-Galactosidase *aus Aspergillus niger* im Gewichtsverhältnis Enzym:Substrat von 1:50 versetzt und gerührt. Der Fortschritt der Transgalactosilierung wurde durch Probennahme verfolgt. Nach etwa 90 Minuten war die maximale GOS-Konzentration erreicht. Der pH-Wert wurde durch Zugabe von 30 Gew.-%iger Natronlauge innerhalb von wenigen Minuten auf 10 erhöht, wodurch die Aktivität des Enzyms schlagartig um 80 % verringert wurde. Der Reaktionsansatz wurde zu einer Ultrafiltrationseinheit geleitet, die mit einer Spiralwickelmembran mit einer Porengröße von 10 kDa versehen war. Das inaktivierte Enzymmaterial wurde mit dem Retentat R1 in den Fermenter zurückgeführt; um Verlust auszugleichen wurden bezogen auf die Ausgangsmenge 5 Gew.-% frisches Enzym zugefügt. Das Permeat P1 wurde zu einer Nanofiltrationseinheit geleitet, die mit einer Keramikmembran mit einer Porenweite von 1.000 Da versehen war. Mit dem Permeat P2 wurden die noch im Produkt enthaltenen Monosaccharide abgetrennt, während das Retentat R2 einer Umkehrosmoseeinheit zugeleitet wurde, die mit einem Konzentrierungsfaktor von 1:2 arbeitete. Das dabei anfallende Permeat P3 (also das Konzentrierungswasser) wurde in das Verfahren zurückgeleitet, das Retentat R3 (also das GOS-Konzentrat) wurde im Plattenwärmeaustauscher 30 Sekunden auf etwa 85 °C erhitzt und über einen Turm versprüht. Es wurde ein weißes Pulver mit einem GOS-Gehalt von mehr als 75 Gew.-% erhalten, welches noch eine Restfeuchte von 1 Gew.-% aufwies. Der DP der GOS betrug etwa 5.

### Herstellbeispiel 3

Den wässrigen Oligosaccharid-Konzentraten aus den Herstellbeispielen 1 und 2 wurde eine solche Menge an Proteinen zugesetzt, dass sich ein Gewichtsverhältnis von Oligosaccharid zu Proteinen von 20:80 ergab. Anschließend wurden die Mischungen auf eine Feststoffkonzentration von 60 Gew.-% verdünnt., im Plattenwärmeaustauscher 30 Sekunden auf etwa 85 °C erhitzt und über einen Turm versprüht. Es wurde ein weißes Pulver erhalten, das noch eine Restfeuchte von 2,2 Gew.-% aufwies.

### Formulierungsbeispiele

Wässrige Lösungen verschiedener Proteine wurden GOS gemäß Herstellbeispiel 1 in unterschiedlichen Mengen versetzt und von 5 Testern bezüglich ihrer Geschmacksqualitäten nachfolgendem Schema beurteilt:
(1) = unangenehm bitter
(2) = bitter
(3) = neutral
(4) = süßlich
(5) = unangenehm süß

Als Standard dienten Lösungen der Proteine ohne Zugabe von Oligosacchariden und Proteinlösungen, die anstelle von GOS Lactose enthielten. Die Ergebnisse sind in **Tabelle 1** zusammengefasst; die Beispiele 1 bis 6 sind erfindungsgemäß, die Beispiele V1 und V2 dienen zum Vergleich.

**Tabelle 1**

| Geschmackliche Beurteilung | | | | | | | | |
|---|---|---|---|---|---|---|---|---|
| **Komponenten** | **1** | **2** | **3** | **4** | **5** | **6** | **V1** | **V2** |
| Kartoffelprotein | 5,0 | 5,0 | 5,0 | - | - | - | - | - |
| Lupinenprotein | - | - | - | 5,0 | 5,0 | 5,0 | 5,0 | 5,0 |
| B-GOS | 0,1 | 0,5 | 1,0 | 0,1 | 0,5 | 1,0 | - | - |
| Lactose | - | - | - | - | - | - | - | 1,0 |
| Wasser | Ad 100 | | | | | | | |
| Geschmackliche Beurteilung | | | | | | | | |
| | 2 | 3 | 4 | 2 | 3 | 4 | 1 | 2 |

### Die Beispiele und Vergleichsbeispiele zeigen:

- GOS vermindern den Bittergeschmack von pflanzlichen Proteinen bereits in kleinen Konzentrationen von 1:50;
- Selbst in hohen Konzentrationen, wie sie in den erfindungsgemäßen Zubereitungen typisch sind, führt die Zugabe von GOS nicht zu einer unangenehmen Süße, sondern nur zu einem süßlichen Geschmack;
- Lactose ist selbst im Einsatzverhältnis 1:20 nicht in der Lage, den bitteren Geschmack der pflanzlichen Proteine zu überdecken.

## Patentansprüche

1. Prebiotische Zubereitungen, enthaltend oder bestehend aus
(a1) Milchproteinen und/oder
(a2) pflanzlichen Proteinen und
(b) Oligosacchariden ausgewählt aus der Gruppe, die gebildet wird von beta-Galactooligosacchariden (β-GOS), alpha-Galactooligosacchariden (α-GOS), Fructooligosacchariden (FOS) und Mannanoligosacchariden (MOS).

2. Zubereitungen nach Anspruch 1, **dadurch gekennzeichnet, dass** die Milchproteine ausgewählt sind aus der Gruppe, die gebildet wird von Casein, Milchproteinkonzentraten, Milchproteinisolaten, Molkenproteinen, Molkenproteinkonzentraten und proteinhaltigen Milchpulvern.

3. Zubereitungen nach den Ansprüchen 1 und/oder 2, **dadurch gekennzeichnet, dass** die pflanzlichen Proteine ausgewählt sind aus der Gruppe, die gebildet wird von Kartoffelproteinen, Sojaproteinen, Erbsenproteinen, Lupinenproteinen, Rapsproteinen, Sonnenblumenproteinen, Kürbiskernproteinen sowie deren Gemischen.

4. Zubereitungen nach mindestens einem der Ansprüche 1 bis 3, **dadurch gekennzeichnet, dass** die beta-Galactooligosaccharide durch Transgalactosilierung von Milchzucker in Gegenwart von beta-Galactosidasen gewonnen sind.

5. Zubereitungen nach mindestens einem der Ansprüche 1 bis 3, **dadurch gekennzeichnet, dass** die alpha-Galactooligosaccharide durch Transgalactosilierung von Rohrzucker in Gegenwart von alpha-Galactosidasen gewonnen sind.

6. Zubereitungen nach mindestens einem der Ansprüche 1 bis 3, **dadurch gekennzeichnet, dass** die Fructooligosaccharide durch enzymatische Prozesse oder Hydrolyse von Inulin gewonnen sind.

7. Zubereitungen nach mindestens einem der Ansprüche 1 bis 3, **dadurch gekennzeichnet, dass** die Mannanoligosaccharide aus Hefezellen gewonnen sind.

8. Zubereitungen nach mindestens einem der Ansprüche 1 bis 7, **dadurch gekennzeichnet, dass** die Oligosaccharide einen DP im Bereich von etwa 5 bis etwa 80 aufweisen.

9. Zubereitungen nach mindestens einem der Ansprüche 1 bis 8, **dadurch gekennzeichnet, dass** sie weiterhin Zusatzstoffe enthalten, die ausgewählt sind aus der Gruppe, die gebildet wird von Stärkeprodukten, Ballaststoffen, Verdickungsmittel, Aromastoffen, weiteren Prebiotika, Probiotika, Genusssäuren, Salzen und Farbstoffen sowie deren Gemischen.

10. Zubereitungen nach mindestens einem der Ansprüche 1 bis 9, **dadurch gekennzeichnet, dass** sie folgende Zusammensetzung aufweisen:
(a) etwa 10 bis etwa 95 Gew.-% Milchproteine und/oder pflanzliche Proteine,
(b) etwa 5 bis etwa 20 Gew.-% Oligosaccharide;
(c1) 0 bis etwa 5 Gew.-% tierische und/oder pflanzliche Fette;
(c2) 0 bis etwa 5 Gew.-% Gew.-% Stärkeprodukte;
(c3) 0 bis etwa 5 Gew.-% Ballaststoffe;
(c4) 0 bis etwa 5 Gew.-% Emulgatoren;
(c5) 0 bis etwa 2 Gew.-% Verdickungsmittel;
(c6) 0 bis etwa 2 Gew.-% Aromastoffe;
(c7) 0 bis etwa 1 Gew.-% probiotische Mikroorganismen;
(c8) 0 bis etwa 1 Gew.-% prebiotische Stoffe;
(c9) 0 bis etwa 1 Gew.-% Genusssäuren,
(c10) 0 bis etwa 1 Gew.-% Süßstoffe;
(c11) 0 bis etwa 1 Gew.-% Antioxidantien und/oder Vitamine;
(c12) 0 bis etwa 1 Gew.-% Lab;
(c13) 0 bis etwa 1 Gew.-% Salze;
(c14) 0 bis etwa 0,5 Gew.-% Farbstoffe;
mit der Maßgabe, dass sich die Mengenangaben gegebenenfalls mit Wasser zu 100 Gew.-% ergänzen.

11. Zubereitungen nach mindestens einem der Ansprüche 1 bis 10, **dadurch gekennzeichnet, dass** sie als wässrige Lösungen oder Dispersionen oder Pulver vorliegen.

12. Verfahren zur Herstellung von prebiotischen Zubereitungen nach Anspruch 1, umfassend oder bestehend aus den folgenden Schritten:
(i) Bereitstellen einer wässrigen Lösung der Proteinkomponente;
(ii) Bereitstellen einer wässrigen Lösung der Oligosaccharid-Komponente;
(iii) Vermischen der beiden Komponenten; gegebenenfalls
(iv) Entwässern der Mischung; sowie gegebenenfalls
(v) Zumischung der weiteren Zusatzstoffe.

13. Verwendung von Zubereitungen nach Anspruch 1 als Nahrungsergänzungsstoffe.

14. Verwendung von Zubereitungen nach Anspruch 1 zur Tierernährung.

15. Verwendung von Oligosacchariden ausgewählt aus der Gruppe, die gebildet wird von beta-Galactooligosacchariden (β-GOS), alpha-Galactooligosacchariden (α-GOS), Fructooligosacchariden (FOS) und Mannanoligosacchariden (MOS) zur Maskierung des bitteren Geschmacks von pflanzlichen Proteinen.
